# EUROPÄISCHE PATENTANMELDUNG

(11) **EP 2 332 494 A1**
(43) Veröffentlichungstag der Anmeldung: **15.06.2011**
(21) Anmeldenummer: 09178484.3
(22) Anmeldetag: 09.12.2009
(51) Int. Cl.: A61F 2/16

(54) **Applikator zur Einführung von Linsen**

(71) Anmelder: Neoptics AG, 6331 Hünenberg (CH)
(72) Erfinder: Berner, Werner, 5018 Erlinsbach (CH); Hauri, Thomas, 5053 Staffelbach (CH)
(74) Vertreter: Welch, Andreas

(57) **Zusammenfassung**

Die vorliegende Erfindung betrifft eine Pre-Load-Einheit (P) zur Einführung von Linsen in das Auge eines Menschen oder Tieres, umfassend ein Gehäuse 2 mit Mitteln zur Befestigung der Einheit an einem Griffstück 7, ein Linsenaufnahmeteil, welches an oder in dem Gehäuse 2 angeordnet ist und einen aus dem Gehäuse 2 herausstehenden Abschnitt mit zwei getrennten blattartigen Einheiten 3 umfasst, welche zumindest an ihren vom Gehäuse 2 entfernten Enden miteinander in lösbarem Kontakt stehen und dort eine Kammer zur Aufbewahrung einer optischen Linse bilden, einen Schieber, welcher beweglich im Innern des Gehäuses 2 angeordnet ist und zwischen den blattartigen Einheiten 3 der Linsenaufnahmeeinheit bewegt werden kann. Die vorliegende Erfindung betrifft weiterhin einen Applikator umfassend eine derartige Pre-Load-Einheit P und ein Griffstück 7, wobei die Pre-Load-Einheit P und das Griffstück 7 derart miteinander verbunden sind, dass die Pre-Load-Einheit P innerhalb des Griffstücks 7 bewegt werden kann.

## Beschreibung

Die vorliegende Erfindung betrifft einen Applikator zum Einführen von optischen Linsen in das Auge eines Menschen oder Tieres.

Bei verschiedenen Massnahmen zur Verbesserung der Sehschärfe des menschlichen Auges ist es üblich, eine optische Linse in das Auge einzuführen. Beispielsweise werden Korrekturlinsen in eine Tasche eingebracht, welche zuvor in der Hornhaut (Cornea) erzeugt wurde (beispielsweise mittels eines Lasers oder eines Keratoms). Zur Einführung der Linsen in diese Taschen wird ein Linseneinführungsgerät (Applikator) verwendet.

Die aus dem Stand der Technik bekannten Applikatoren funktionieren derart, dass eine vom Applikator gehaltene Linse aus dem Applikator heraus in die Hornhauttasche gestossen wird. Derartige Applikatoren sind beispielsweise in der WO 2006/007543 und in der US-5,123,905 gezeigt.

Die ausgestossenen Linsen müssen sich teilweise in der Tasche erst noch entfalten und sind zudem in aller Regel in der Tasche noch genau zu positionieren. Die eingesetzten Linsen sind sehr flexibel und nur schwer zu handhaben.

Es bestand daher das Bedürfnis nach einem Applikator, mit welchen optische Linsen auf einfachere und exaktere Art in das Auge eines Menschen oder Tieres eingebracht werden können.

Die vorstehende Aufgabe wird durch den erfindungsgemässen Applikator gelöst.

Die vorstehende Erfindung beruht auf einem völlig anderen Prinzip der Einführung einer Linse in das Auge: Statt wie im Stand der Technik die Linse mit Hilfe eines herkömmlichen Applikators in das Innere des Auges hineinzustossen, wird bei dem erfindungsgemässen Applikator die Linse bereits korrekt im Auge positioniert, während sie sich noch innerhalb des Applikators befindet. Anschliessend wird der Applikator unter Erhalt der Linsenposition um einen durch das Griffstück definierten Betrag zurückgezogen und so die Linse an der richtigen Position freigegeben. Anschliessend wird der Applikator aus der Hornhauttasche zurückgezogen.

Auf diese Weise ist eine viel einfachere und exaktere Positionierung der Linse an der gewünschten Stelle im Auge möglich. Anstatt die flexible und schwer handhabbare Linse nach Ausstoss in das Auge zu positionieren, wird vielmehr der leichter handhabbare Applikator exakt ausgerichtet. Anschliessend wird der Applikator entfernt, während die Linse an Ort und Stelle positioniert bleibt.

Dies wird durch eine zweiteilige Ausgestaltung des erfindungsgemässen Applikators erreicht. Der erfindungsgemässe Applikator umfasst eine mit einer Linse bestückbare Pre-Load-Einheit und ein Griffstück. Pre-Load-Einheit und Griffstück können so miteinander verbunden werden, dass die Pre-Load-Einheit um einen definierten Betrag zurückgezogen werden kann und der Schieber in Position bleibt. Dies gelingt durch eine vorgängige Fixierung der Linse mit Hilfe eines unabhängig von der restlichen Pre-Load-Einheit bewegbaren Schiebers. Die Pre-Load-Einheit ist vorzugsweise mit einer Verdrehsicherung ausgestaltet, sodass die Orientierung der Linse auch beim Aufsetzen auf das Griffstück erhalten bleibt.

Gemäss der vorliegenden Erfindung wird unter dem Zurückziehen um einen definierten Betrag verstanden, dass das entsprechende Bauteil um eine bestimmte Länge in das Griffstück hineingezogen werden kann. Die Länge wird dabei durch die Beschaffenheit des Griffstücks vorgegeben, muss aber mindestens derart sein, dass die in der Pre-Load-Einheit enthaltene Linse aus der Pre-Load-Einheit freigesetzt werden kann.

Ein Aspekt der vorliegenden Erfindung betrifft eine Pre-Load-Einheit zur Einführung von Linsen in das Auge eines Menschen oder Tieres, umfassend
i) ein Gehäuse mit Mitteln zur, vorzugsweise verdrehsicheren, Befestigung der Einheit an einem Griffstück,
ii) ein Linsenaufnahmeteil, welches an oder in dem Gehäuse angeordnet ist und einen aus dem Gehäuse herausstehenden Abschnitt mit zwei getrennten blattartigen Einheiten umfasst, welche zumindest an ihren vom Gehäuse entfernten Enden miteinander in lösbarem Kontakt stehen und dort eine Kammer zur Aufbewahrung einer optischen Linse bilden,
iii) einen Schieber, welcher beweglich im Innern des Gehäuses angeordnet ist und zwischen den blattartigen Einheiten der Linsenaufnahmeeinheit bewegt werden kann.

Gemäss der vorliegenden Erfindung soll unter einer blattartigen Einheit ein Bauteil verstanden werden, welches analog zu einem Blatt sehr dünn ist und damit eine entsprechende Flexibilität aufweist. Gemäss der vorliegenden Erfindung sind die blattartigen Einheiten typischerweise zwischen 0,1 und 0,3 mm dick und typischerweise 20-40, vorzugsweise 20 bis 30 mm lang.

Gemäss einer bevorzugten Ausführungsform weisen die zwei blattartigen Einheiten jeweils mindestens ein, vorzugsweise genau ein Loch auf, welche so angeordnet sind, dass die Löcher direkt übereinander liegen und eine Öffnung durch das Zentrum der Kammer zur Aufbewahrung einer optischen Linse bilden. Auf diese

Weise ist es besonders gut möglich, eine in der Kammer befindliche Linse auf der Sehachse des Auges zu positionieren. Die das Einsetzen der Linse vornehmende Person sieht die Linse durch die Öffnung und kann sie exakt ausrichten. Diese Löcher können typischerweise einen Durchmesser von 0,3 bis 0,6 mm aufweisen. Diese Ausführungsform ist besonders für Intracorneallinsen mit einem zentralen Loch vorteilhaft, wie sie in der WO 2009/075685 beschrieben sind.

Die vorliegende Erfindung umfasst gemäss ihrer besonders bevorzugten Ausführungsformen eine Pre-Load-Einheit mit genau zwei derartigen blattartigen Einheiten. Allerdings ist es aber auch möglich, Pre-Load-Einheiten mit mehr als zwei, beispielsweise vier blattartigen Einheiten zu verwenden, wobei die blattartigen Einheiten zusammen eine Kammer zur Aufbewahrung einer optischen Linse bilden.

Wie vorstehend beschrieben bilden die blattartigen Einheiten des Linsenaufnahmeteils an ihren vom Gehäuse entfernten Enden eine Kammer zur Aufbewahrung einer optischen Linse. Diese Kammer muss geöffnet werden können, um die Linse in die Kammer einzuführen beziehungsweise wieder aus der Kammer zu entlassen. Gemäss der vorliegenden Erfindung befinden sich die blattartigen Einheiten daher im Bereich der Kammer in losem Kontakt miteinander, d.h. sie können durch Kraftanwendung voneinander weg bewegt werden. Die blattartigen Einheiten können beispielsweise mittels des nachstehend näher beschriebenen Schiebers auseinander gespreizt werden. Zur Lösung des Kontakts der blattartigen Einheiten ist aber eine derartige Kraftanwendung erforderlich.

Um die Linse innerhalb der Kammer an Ort und Stelle zu halten, wird die Kammer zumindest teilweise durch wellenförmige Abschnitte begrenzt, welche auf beiden blattartigen Einheiten vorhanden und so angeordnet sind, dass die Abschnitte der einen blattartigen Einheit mit den entsprechenden Abschnitten der anderen blattartigen Einheit verzahnt sind. Auf diese Weise wird eine Kammer gebildet, welche quasi "vergittert" ist. Besonders bevorzugt wird durch diese Begrenzungen eine Kammer gebildet, in welche die in das Auge einzuführende Line eingebracht werden kann.

Erfindungsgemäss bevorzugt sind diese Begrenzungen derart auf den blattartigen Einheiten angeordnet, dass im Zustand des Kontakts der beiden blattartigen Einheiten ein Verrutschen der Linse aus der Kammer nicht möglich ist und sie in der Kammer beweglich ist (zum Beispiel indem die Begrenzungen an allen Kammerecken vorgesehen sind). Vorzugsweise sind die wellenförmigen Abschnitte zusätzlich etwas erhöht.

Wie vorstehend ausgeführt besteht das grundlegend neue Prinzip der vorliegenden Erfindung darin, nicht die Linse in eine Hornhauttasche zu stossen, sondern vielmehr den Applikator in der Hornhauttasche exakt zu positionieren und anschliessend unter Orterhalt der Linse die blattartigen Teile um einen genau definierten Betrag zurückzuziehen. Daher muss der Abschnitt des Applikators (beziehungsweise der Pre-Load-Einheit des Applikators), in welchem die Kammer zur Aufbewahrung einer optischen Linse ausgebildet ist, derart geformt sein, dass er in eine entsprechende Hornhauttasche eingeführt werden kann. Erfindungsgemäss bevorzugt entspricht die Form des diese Kammer bildenden Bereichs des Applikators im wesentlichen der Form der zu platzierenden Linse beziehungsweise ist in seiner Breite sogar geringfügig kleiner als die entsprechende Linse. Dies ist zum Beispiel dadurch möglich, dass die Kammer keine runde Form hat und nicht überall die vorstehend beschriebenen Begrenzungen aufweist, d.h. zwischen den Begrenzungen ist die Kammer seitlich offen. Die in der Kammer befindliche Linse kann dann an einigen Stellen aus der Kammer herausragen.

Erfindungsgemäss bevorzugt weisen die blattartigen Einheiten der Pre-Load-Einheit im Bereich der Kammer eine Breite von 3 mm oder etwas weniger auf. Dies kann aber in Abhängigkeit der zu platzierenden Linse variieren.

Um eine Öffnung der Kammer zu ermöglichen, müssen die blattartigen Einheiten voneinander weggespreizt werden können. Sie müssen daher aus einem ausreichend flexiblen Material gefertigt sein. Gemäss der vorliegenden Erfindung kommt als Material für die blattartigen Einheiten ein physiologisch verträglicher Kunststoff oder ein physiologisch verträgliches Metall in Frage.

Die blattartigen Einheiten sind typischerweise 20-40, vorzugsweise 20 bis 30 mm lang. Vorzugsweise gehen sie unter zunehmender Verdickung in einen Abschnitt des Linsenaufnahmeteils über, welcher fest in dem Gehäuse der Pre-Load-Einheit angeordnet ist. Alternativ können die blattartigen Einheiten beispielsweise aber auch eine gleich bleibende Dicke aufweisen und an einem Abschnitt des Linsenaufnahmeteils befestigt sein, welcher fest in dem Gehäuse der Pre-Load-Einheit angeordnet ist. Zwischen den blattartigen Einheiten befindet sich aber auf jeden Fall ein ausreichend grosser Zwischenraum, damit der nachstehend erläuterte Schieber darin frei bewegt werden kann. Weiterhin sind die blattartigen Einheiten auf jeden Fall derart an dem weiteren Abschnitt des Linsenaufnahmeteils oder sogar direkt am Gehäuse befestigt, dass sie sich an ihren vom Gehäuse entfernten Enden berühren und dort die vorstehend beschriebene Kammer ausbilden.

Das Gehäuse der Pre-Load-Einheit dient zur Fixierung der Linsenaufnahmeeinheit. Erfindungsgemäss bevorzugt wird die Linsenaufnahmeeinheit in das Gehäuse hinein geführt und dort durch geeignete Verankerungsmechanismen arretiert. Alternativ kann die Linsenaufnahmeeinheit aber auch beispielsweise fest an dem Gehäuse angeordnet sein.

Das Gehäuse der Pre-Load-Einheit besitzt eine durchgehende Bohrung, in welcher sich der nachstehend näher erläuterte Schieber frei bewegen kann.

Die Pre-Load-Einheit ist für den Einsatz in einem erfindungsgemässen Applikator vorgesehen. Zu diesem Zweck weist das Gehäuse der Pre-Load-Einheit Mittel zur Befestigung der Einheit an einem Griffstück auf. Diese Mittel sind an dem Ende des Gehäuses angeordnet, welches dem Ende mit den herausstehenden blattartigen Einheiten entgegengesetzt ist. Vorzugsweise ist die Verbindung mit dem Griffstück so ausgestaltet, dass sie nur verdrehsicher erfolgen kann.

Erfindungsgemäss bevorzugt handelt es sich bei den Befestigungsmitteln um Mittel zur Herstellung eines Bajonettverschlusses mit dem Griffstück. Es sind selbstverständlich aber auch andere geeignete Befestigungsmittel einsetzbar.

Wie vorstehend ausgeführt besteht das erfindungsgemässe Prinzip darin, die Pre-Load-Einheit nach erfolgter Positionierung in der Hornhauttasche unter Orterhalt der Linse um einen definierten Betrag zurückzuziehen. Zu diesem Zweck muss die Pre-Load-Einheit in das Griffstück hinein bewegbar sein. Dies wird nachstehend näher ausgeführt. Erfindungsgemäss bevorzugt weist die Pre-Load-Einheit aber einen Stopper zur Begrenzung der Bewegung der Pre-Load-Einheit in das Griffstück hinein auf. Es kann sich hierbei beispielsweise um eine Erhöhung an einer Stelle der Oberfläche des Gehäuses handeln. Vorzugsweise ist diese Erhöhung ringförmig um den gesamten Umfang des Gehäuses ausgebildet. Der Stopper ist an einer Stelle des Gehäuses der Pre-Load-Einheit angeordnet, dass das Gehäuse zumindest soweit in das Griffstück hinein bewegt werden kann, dass die blattartigen Einheiten nicht mehr die in der Kammer befindliche Linse bedecken, d.h. dass die Linse freigelegt ist.

Erfindungsgemäss bevorzugt ist die Pre-Load-Einheit für den Einmalgebrauch konzipiert. Die Pre-Load-Einheit wird demnach einmal mit einer entsprechenden Linse beschickt und anschliessend steril verpackt, ehe sie kurz vor dem Einsetzprozess aus der Verpackung geholt und mit dem Griffstück verbunden wird.

Um die Pre-Load-Einheit mit einer Linse zu beschicken, werden die blattartigen Einheiten voneinander weg gespreizt, um einen Zugang zur vorstehend beschriebenen Kammer zu ermöglichen. Gemäss einer Ausführungsform der vorliegenden Erfindung kann man hierfür ein geeignetes Werkzeug, beispielsweise ein Messer, in den seitlichen Zwischenraum zwischen den blattartigen Einheiten einführen. Anschliessend wird die Linse, vorzugsweise unter einem Mikroskop, in die zugängliche Kammer platziert. Anschliessend wird das Werkzeug aus dem seitlichen Zwischenraum zwischen den blattartigen Einheiten entfernt, wodurch die blattartigen Einheiten an ihren Enden wieder in Kontakt zueinander kommen und die Kammer geschlossen wird. Für den Beladungsvorgang kann man beispielsweise eine Beladungsstation bereitstellen, in welcher die Pre-Load-Einheit fixiert werden kann. Die Beladungsstation weist ein drehbares Werkzeug (z.B. ein Messer) auf, welches in den seitlichen Zwischenraum zwischen den blattartigen Einheiten hineingedreht und aus diesem wieder herausgedreht werden kann.

Erfindungsgemäss bevorzugt weist zumindest eine blattartige Einheit ein weiteres Loch auf, welches oberhalb der Begrenzung der

Kammer zur Aufbewahrung einer optischen Linse angeordnet ist. Dies ermöglicht beim vorstehenden Beladungsvorgang eine bessere Kontrolle der korrekten Platzierung der Linse in der Kammer, da die den Vorgang ausführende Person durch das Loch erkennen kann, dass die Linse exakt in der Kammer angeordnet ist und nicht beispielsweise auf einer der Begrenzungen liegt. Dieses zweite Loch kann typischerweise einen Durchmesser von 0,3 bis 0,6 mm aufweisen.

Ein wesentliches Element der erfindungsgemässen Pre-Load-Einheit ist ein Schieber, welcher beweglich im Innern des Gehäuses angeordnet ist und zwischen den blattartigen Einheiten der Linsenaufnahmeeinheit bewegt werden kann. Dieser Schieber muss zwischen den blattartigen Einheiten so weit nach vorne bewegt werden können, dass er in Kontakt mit der in der Kammer befindlichen Linse kommt und diese fixieren kann. Vorzugsweise verjüngt sich der Schieber zu dem zwischen den blattartigen Einheiten zu bewegenden Ende hin. Zur besseren Fixierung der Linse ist das vordere Ende des Schiebers entsprechend komplementär zur Linsenform ausgebildet.

Innerhalb der des Gehäuses ist der Schieber frei und unabhängig von den anderen Bauteilen beweglich. Vorzugsweise weist der im Gehäuse der Pre-Load-Einheit befindliche Abschnitt des Schiebers eine Dicke knapp unterhalb des Durchmessers der durchgehenden Bohrung im Gehäuse der Pre-Load-Einheit auf. Innerhalb dieser Bohrung ist der Schieber für Bedienelemente des nachstehend beschriebenen Griffstücks zugänglich.

Wie vorstehend ausgeführt ist die Pre-Load-Einheit vorzugsweise für den Einmalgebrauch konzipiert. Gemäss einem weiteren Aspekt der vorliegenden Erfindung wird daher die Pre-Load-Einheit in einer sterilen Verpackung gelagert, aus welcher sie erst unmittelbar vor dem Einsetzen der Linse in das Auge entnommen und mit dem Griffstück verbunden wird.

Die vorliegende Erfindung betrifft somit auch ein Kit, umfassend eine Aufbewahrungseinheit und eine vorstehend beschriebene Pre-Load-Einheit im Innern der Aufbewahrungseinheit, wobei die Aufbewahrungseinheit aus einem wasserdichten Material besteht und mit einem Stopfen wasserdicht verschliessbar ist.

Besonders bevorzugt wird die Pre-Load-Einheit mit einer gewünschten optischen Linse beschickt und anschliessend steril verpackt. Dies erleichtert die Tätigkeit der die Linse einsetzenden Person.

Um die Pre-Load-Einheit über eine längere Zeit steril lagern zu können, wird sie in einer Aufbewahrungseinheit verpackt, welche die Pre-Load-Einheit vor Umwelteinflüssen schützt. Zu diesem Zweck ist die Aufbewahrungseinheit im Innern mit einer Lagerflüssigkeit gefüllt, welche mindestens die in der Kammer der Pre-Load-Einheit befindliche Linse ständig bedeckt. Es kann sich dabei um Wasser handeln; bevorzugt ist aber physiologische Kochsalzlösung (NaCl) als Lagerflüssigkeit.

Die Aufbewahrungseinheit ist aus einem Material gefertigt, welches wasserdicht ist und somit auch Wasserdampf wenn überhaupt nur in sehr geringem Mass passieren lässt. Dies ist wichtig, um einen ausreichenden Pegel an Lagerflüssigkeit innerhalb der Aufbewahrungseinheit während der gesamten Lagerungszeit zu gewährleisten. Bei nicht wasserdichtem Material würde die Lagerflüssigkeit über einen gewissen Zeitraum verdampfen, und die Linse in der Kammer der Pre-Load-Einheit wäre nicht mehr steril gelagert. Wasserdichte Materialien sind dem Fachmann bekannt. Als Beispiel seien Glass oder ein wasserdichter Kunststoff genannt. Nach Einbringen der Pre-Load-Einheit wird die Aufbewahrungseinheit wasserdicht mit einem geeigneten Stopfen verschlossen. Dieser Stopfen ist vorzugsweise aus einem wasserdichten Kunststoff gefertigt.

Der erfindungsgemässe Applikator umfasst weiterhin ein Griffstück. Typischerweise handelt es sich hierbei um ein längliches Rohr mit einer Form, welche ein einfaches Halten des Griffstücks in der Hand gewährleistet. Wie vorstehend ausgeführt ist das Griffstück an einem Ende derart ausgebildet, dass die Pre-Load-Einheit und das Griffstück derart miteinander verbunden werden können, dass die Pre-Load-Einheit innerhalb des Griffstücks bewegt werden kann. Hierzu weist das Griffstück einen Durchmesser auf, welcher den Durchmesser des Teils der Pre-Load-Einheit übersteigt, welcher in das Griffstück einzuführen ist. Zumindest an dem mit der Pre-Load-Einheit zu verbindenden Ende ist das Griffstück innen hohl. Vorzugsweise handelt es sich aber um ein vollständig hohles Rohr.

Die Pre-Load-Einheit wird mit Teilen innerhalb des Griffstücks verbunden, welche beweglich sind. Durch eine Bewegung dieser Teile wird somit auch die Pre-Load-Einheit bewegt. Vorzugsweise wird diese Bewegung mit Hilfe eines Bedienelements vorgenommen, welches sich auf der Oberfläche des Griffstücks befindet. Vorzugsweise handelt es sich bei dem Bedienelement um einen Schieberegler, welcher durch eine längliche Öffnung im Griffstück mit den entsprechenden Teilen im Innenraum des Griffstücks verbunden ist.

Gemäss der vorliegenden Erfindung ist der Schieber der Pre-Load-Einheit unabhängig von der restlichen Pre-Load-Einheit zu bewegen. Erfindungsgemäss bevorzugt wird dies durch ein weiteres Bedienelement erreicht, welches sich ebenfalls auf der Oberfläche des Griffstücks befindet. Vorzugsweise handelt es sich auch bei diesem Bedienelement um einen Schieberegler, welcher durch eine längliche Öffnung im Griffstück mit entsprechenden Einlegteilen im Innenraum des Griffstücks verbunden ist. Diese Einlegteile ähneln dem Ausstosser eines Gewehrs. Wird das Bedienelement auf der Oberfläche des Griffstücks nach vorne bewegt, kommen die Einlegteile in Kontakt mit dem Schieber der Pre-Load-Einheit und bewegen diesen unabhängig von der restlichen Pre-Load-Einheit nach vorne.

Um diese Einlegteile im Griffstück zu fixieren, ist gemäss einer bevorzugten Ausgestaltung der vorliegenden Erfindung an den Einlegteilen ein Stift angebracht, welcher durch eine Öffnung im Griffstück ragt und dort fixiert ist.

Gemäss einer besonders bevorzugten Ausführungsform der vorliegenden Erfindung sind die beiden vorstehend beschriebenen Bedienelemente derart miteinander verbunden, dass das zweite Bedienelement, welches die Pre-Load-Einheit mit Ausnahme des Schiebers bewegt, nur bedient werden kann, wenn das erste Bedienelement, welches den Schieber der Pre-Load-Einheit bewegt, sich in einer Position befindet, in welcher der damit bewegte Schieber maximal aus dem Gehäuse der Pre-Load-Einheit herausgefahren ist. Ansonsten ist das zweite Bedienelement arretiert. Auf diese Weise wird ein unbeabsichtigtes Bewegen der blattartigen Einheiten der Pre-Load-Einheit ohne vorgängige ortserhaltende Fixierung der Linse verhindert.

Das Griffstück des erfindungsgemässen Applikators ist für eine mehrfache Anwendung vorgesehen. Zu diesem Zweck ist das Griffstück gemäss einer bevorzugten Ausführungsform der vorliegenden Erfindung vollständig demontierbar, so dass sämtliche Bauteile vollständig gereinigt und sterilisiert werden können. Die auf der Oberfläche angeordneten Bedienelemente können gemäss dieser Ausführungsform entfernt werden, wodurch die mit den Bedienelementen verbundenen Bauteile im Innern des Griffstücks aus dem Griffstück herausgeholt werden können. Im Fall der zur Bewegung des Schiebers vorgesehenen Einlegteile muss zusätzlich der vorstehend beschriebene Stift aus der Öffnung gedrückt werden, um die Einlegteile entnehmen zu können.

Nach erfolgter Reinigung kann das Griffstück wieder zusammengesetzt werden, wobei darauf zu achten ist, dass sich die Bedienelemente in der korrekten Position befinden.

Gemäss einer alternativen Ausführungsform der vorliegenden Erfindung ist es aber auch möglich, das Griffstück ebenfalls für den Einmalgebrauch vorzusehen. In dieser Ausführungsform ist es entsprechend nicht erforderlich, dass das Griffstück zu Reinigungszwecken demontierbar ist. Gemäss dieser Ausführungsform wird das Griffstück vorzugsweise separat ebenfalls in einer Aufbewahrungseinheit analog der vorstehend beschriebenen Einheit bis zur Verwendung aufbewahrt. Es ist gemäss dieser Ausführungsform aber auch denkbar, dass die Pre-Load-Einheit und das Griffstück bereits als Einheit (entweder wie vorstehend beschrieben trennbar oder alternativ auch fest miteinander verbunden) in einer Aufbewahrungseinheit analog der vorstehend beschriebenen Einheit bis zur Verwendung aufbewahrt werden. Der Applikator gemäss dieser alternativen Ausführungsform kann daher anders aufgebaut sein, indem die vorstehenden Teile zur Verbindung von Griffstück und Pre-Load-Einheit durch eine feste Verbindung dieser Bauteile ersetzt sein können.

Mit Hilfe des erfindungsgemässen Applikators ist eine einfachere und exaktere Positionierung einer Linse im Auge möglich. Vorzugsweise handelt es sich hierbei um die korrekte Positionierung einer Intracorneallinse in einer Hornhauttasche.

Gemäss eines weiteren Aspekts betrifft die vorliegende Erfindung auch ein Verfahren zur Einführung einer optischen Linse, vorzugsweise einer Intracorneallinse, in das Auge eines Menschen oder Tieres, umfassend die Schritte
a) Positionieren des vorstehend beschriebenen Applikators an der gewünschten Stelle des Auges, vorzugsweise in einer Tasche in der menschlichen Cornea, so dass das Zentrum der im Applikator enthaltenen optischen Linse auf der Sehachse des Auges liegt;
b) Vorschieben des Schiebers mittels eines ersten Bedienelements am Griffstück, bis der Schieber in Kontakt mit der Linse kommt ohne diese zu bewegen, wobei gleichzeitig die blattartigen Einheiten des Linsenaufnahmeteils voneinander weg gespreizt werden;
c) Zurückziehen der restlichen Pre-Load-Einheit um einen definierten Betrag mittels eines zweiten Bedienelements am Griffstück unter gleichzeitiger Fixierung des Schiebers, wodurch die Linse aus dem Applikator freigesetzt wird.

Die vorzugsweise steril gelagerte Pre-Load-Einheit wird aus dem Aufbewahrungsbehälter entnommen und mit dem Griffstück vorzugsweise verdrehsicher verbunden. Anschliessend wird der so erhaltene Applikator mit der bestückten Linse an der gewünschten Stelle des Auges positioniert. Vorzugsweise werden die Enden der blattartigen Einheiten der Pre-Load-Einheit, welche die Kammer mit der darin befindlichen Linse bilden, in einer zuvor erzeugte Tasche in der Hornhaut eines Auges eingeführt. Gemäss einer besonders bevorzugten Ausführungsform der vorliegenden Erfindung weist die Pre-Load-Einheit eine durch die Kammer mit der Linse gehende Öffnung auf. Dies ermöglicht der das Verfahren durchführenden Person, die Linse exakt auf der Sehachse des Auges auszurichten, während sich die Linse noch in der Kammer des Applikators befindet. Dies kann erfindungsgemäss viel einfacher durchgeführt werden, weil der Applikator deutlich einfacher handhabbar ist als eine einzelne, kleine und flexible Linse.

Anschliessend wird die Linse innerhalb des Applikators fixiert, indem das erste Bedienelement am Griffstück vollständig nach vorne geschoben wird, wodurch der Schieber der Pre-Load-Einheit maximal nach vorne bewegt wird, so dass er in Kontakt mit der in der Kammer befindlichen Linse kommt. Dies wird dadurch möglich, weil durch das Vorwärtsbewegen des Schiebers die blattartigen Einheiten voneinander weg gespreizt werden und somit das Innere der Kammer zugänglich wird.

Im nächsten Schritt wird nun die restliche Pre-Load-Einheit ausgenommen vom Schieber mit Hilfe des zweiten Bedienelements vom Auge weg um einen definierten Betrag zurückgezogen, indem die restliche Pre-Load-Einheit in das Griffstück hinein gezogen wird. Während dieses Schritts bleibt der Schieber fixiert und hält somit die Linse exakt an der zuvor ausgerichteten Position.

Durch das Zurückziehen der restlichen Pre-Load-Einheit wird die Linse aus dem Applikator exakt in der zuvor ausgerichteten Position freigesetzt. Die Linse ist somit bereits wie gewünscht im Auge platziert. Eines weiteren Ausrichtungsschrittes bedarf es gemäss der vorliegenden Erfindung nicht mehr.

Anschliessend wird der Applikator vom Auge entfernt und in seine Einzelteile zerlegt. Vorzugsweise wird die für den Einmalgebrauch vorgesehene Pre-Load-Einheit nun entsorgt, während das Griffstück zerlegt und gereinigt wird, so dass es für den nächsten Einsatz mit einer neuen Pre-Load-Einheit zur Verfügung steht.

Die vorliegende Erfindung ist vor allem für die Einführung von optischen Linsen, insbesondere Intracorneallinsen in Hornhauttaschen vorgesehen. Grundsätzlich kann aber der erfindungsgemässe Applikator dazu benutzt werden, auch andere Gegenstände als Linsen in das Auge einzuführen.

Als Beispiel sei die Einführung von Gewebe ("Spenderhornhaut") im DSAEK-Verfahren (DESCEMET-ABTRAGENDE AUTOMATISIERTE ENDOTHEL-KERATOPLASTIK (DESCEMET STRIPPING AUTOMATED ENDOTHELIAL KERA-TOPLASTY)) genannt. Dieses Verfahren wird bei einer Beschädigung des Endothels (der hinteren Oberfläche der Hornhaut) eingesetzt, um eine dadurch verursachte Hornhaut-Blindheit zu behandeln (Übersicht in Cursiefen/Kruse, Ophthalmologe 2008, 105, 183-192).

Beim DSAEK-Verfahren wird nicht die gesamte Hornhaut ersetzt, sondern vorteilhaft nur die erkrankte Hornhautschicht. Es wird mit Hilfe eines Keratoms (wie beispielsweise in der EP-1 778 141 A1) beschrieben) ein kleiner Schnitt in die äußere Schicht der Hornhaut gemacht. Anschliessend wird die Hornhaut freigelegt und der trüb gewordene Bereich durch eine Spenderhornhaut ersetzt.

Die Spenderhornhautschicht kann hierbei mit Hilfe des erfindungsgemässen Applikators eingeführt werden. Anstelle einer Linse enthält die Kammer der Pre-Load-Einheit bei dieser Variante eine Spenderhornhaut, welche analog wie vorstehend für eine Linse beschrieben in die Hornhauttasche appliziert werden kann. Grundsätzlich sind keine weiteren Anpassungen des Applikators beziehungsweise des mit dem Applikator durchgeführten Verfahrens erforderlich.

Die vorliegende Erfindung wird nachstehend anhand von nicht einschränkenden Figuren und Beispielen näher erläutert.

Es zeigen:
- Fig.1: eine Ausführungsform des erfindungsgemässen Applikators
- Fig.2: eine Ausführungsform der erfindungsgemässen Pre-Load-Einheit
- Fig. 3a: eine Ausführungsform des vorderen Abschnitts der erfindungsgemässen Pre-Load-Einheit
- Fig. 3b: Der vordere Abschnitt der erfindungsgemässen Pre-Load-Einheit gemäss Fig. 3a in geöffnetem Zustand
- Fig. 4: einen Schnitt durch eine Ausführungsform der erfindungsgemässen Pre-Load-Einheit
- Fig. 5: eine Ausführungsform des erfindungsgemässen Kits aus Aufbewahrungseinheit und Pre-Load-Einheit
- Fig. 6: die Freisetzung der Linse aus der erfindungsgemässen Pre-Load-Einheit

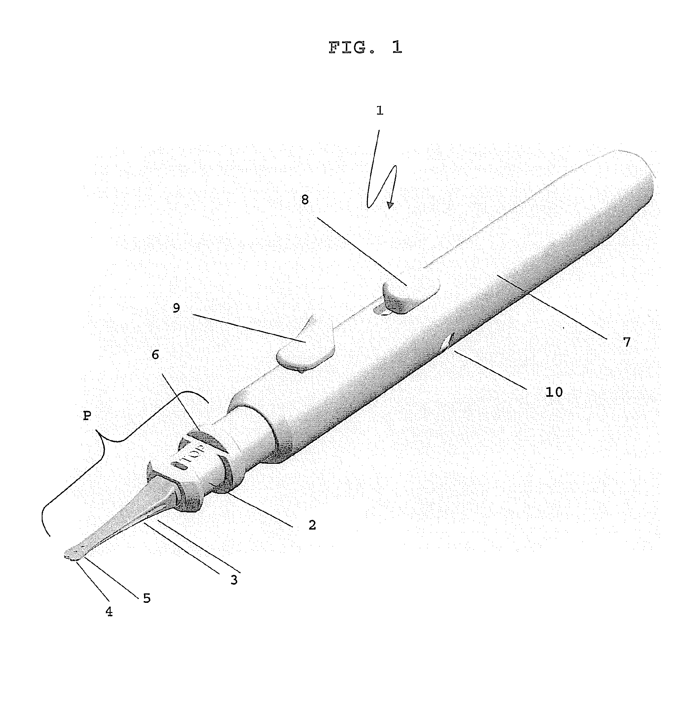

In **Fig. 1** ist eine Ausführungsform des erfindungsgemässen Applikators 1 gezeigt. In dem Griffstück 7 ist eine Pre-Load-Einheit P beweglich angebracht. Die Pre-Load-Einheit P umfasst ein Gehäuse 2, zwei blattartige Einheiten 3 und einen Stopper 6. In den blattartigen Einheiten 3 ist eine durchgehende Öffnung 4 durch das Zentrum der (hier nicht erkennbaren) Kammer zur Aufnahme einer Linse vorhanden. Zudem ist ein weiteres Loch 5 bereitgestellt, welches das Beschicken des Applikators 1 mit einer Linse vereinfacht. Auf dem Griffstück 7 sind zwei Bedienelemente 8 und 9 angebracht. Aus der seitlichen Öffnung 10 ragt ein Stift heraus, welcher an den im Griffstück vorhandenen Einlegeteilen befestigt ist und diese im Griffstück 7 fixiert.

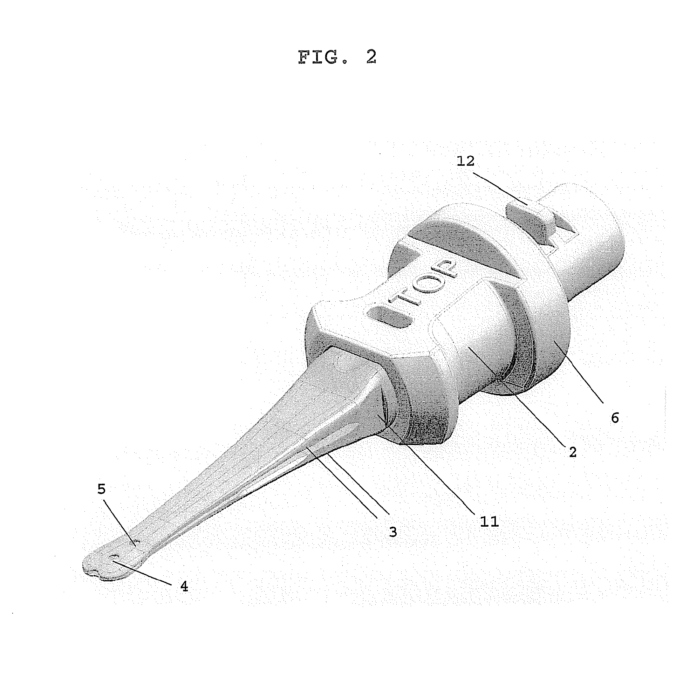

In **Fig. 2** ist die Pre-Load-Einheit P aus Fig. 1 separiert und näher erläutert. Im Gehäuse 2 ist das Linsenaufnahmeteil 11 arretiert. Das Linsenaufnahmeteil 11 steht aus dem Gehäuse 2 heraus und geht in die zwei blattartigen Einheiten 3 über. Am anderen Ende des Gehäuses 2 befinden sich Befestigungsmittel 12, um die Pre-Load-Einheit P mit dem Griffstück 7 zu verbinden. Im vorliegenden Fall ist eine Bajonettverbindung realisiert.

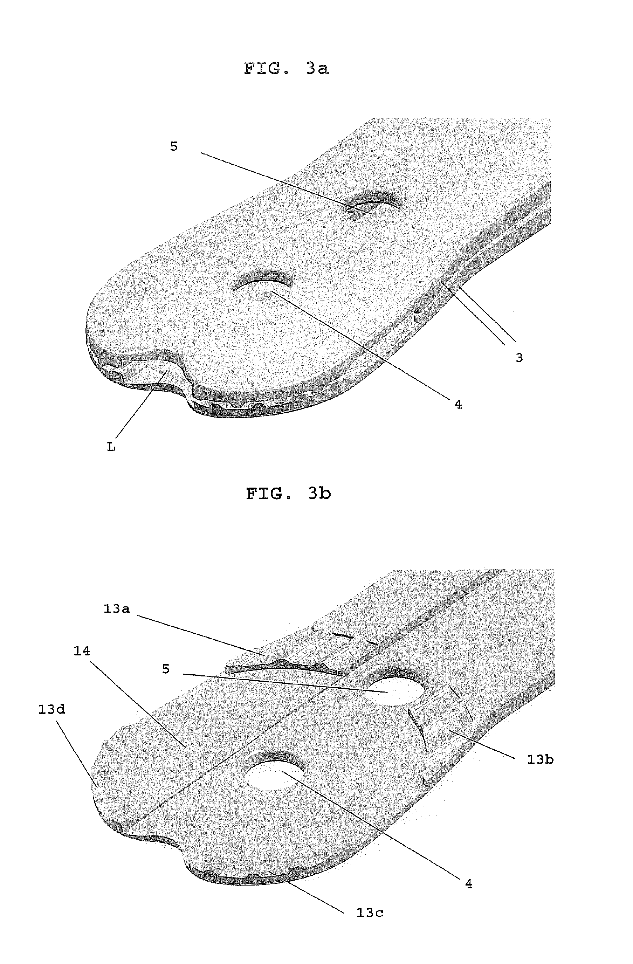

In **Fig. 3a** ist der vordere Abschnitt der erfindungsgemässen Pre-Load-Einheit P im Detail gezeigt. In **Fig. 3b** ist der gleiche vordere Abschnitt in geöffnetem Zustand (ohne die obere blattartige) Einheit 3 gezeigt. Die Linse L befindet sich in der Kammer 14 zwischen den blattartigen Einheiten 3. Die blattartigen Einheiten 3 weisen Begrenzungen 13a-13d um die Kammer 14 auf. Diese Begrenzungen 13a-13d sind wellenförmig ausgebildet und greifen verzahnend ineinander ein. Sie sind etwas erhöht und bilden somit eine Kammer 14, in welche eine Linse L eingebracht werden kann.

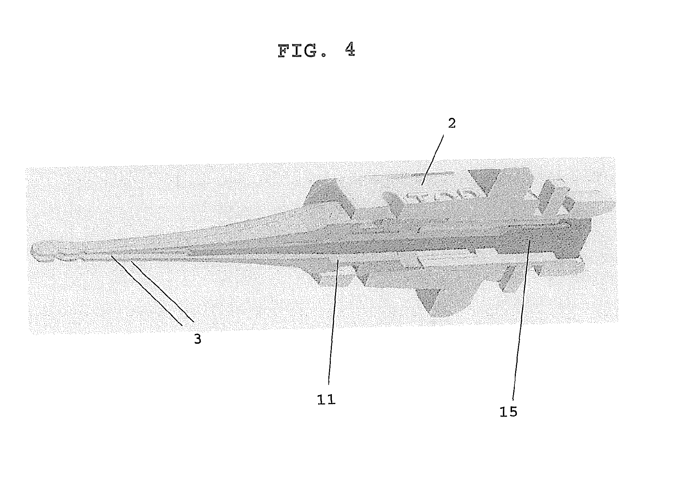

In **Fig. 4** ist ein Schnitt durch eine Ausführungsform der erfindungsgemässen Pre-Load-Einheit P gezeigt. Das Gehäuse 2 weist im Innern eine durchgehende Bohrung auf, in welcher das Linsenaufnahmeteil 11 arretiert ist. Ein Schieber 15 ist ebenfalls im Innern des Gehäuses 2 angeordnet und dort frei beweglich. Nach vorne verjüngt sich der Schieber 15, so dass er zwischen den blattartigen Einheiten 3 bis zur Kammer mit der dort befindlichen Linse L bewegt werden kann.

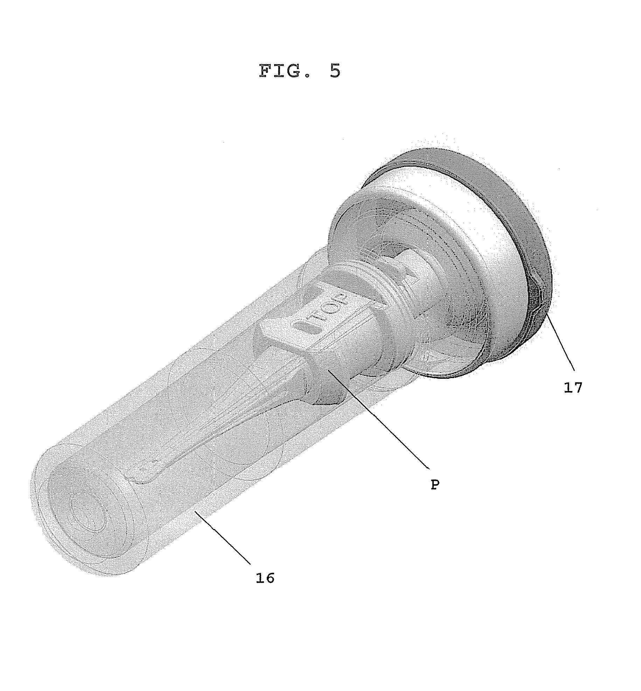

In **Fig. 5** ist eine Ausführungsform des erfindungsgemässen Kits gezeigt, welches eine Aufbewahrungseinheit 16 umfasst. Im Innern der Aufbewahrungseinheit 16 ist die Pre-Load-Einheit P in einer physiologischen Kochsalzlösung gelagert. Die Aufbewahrungseinheit 16 ist aus Glas. Sie ist mit einem Stopfen 17 wasserdicht verschlossen.

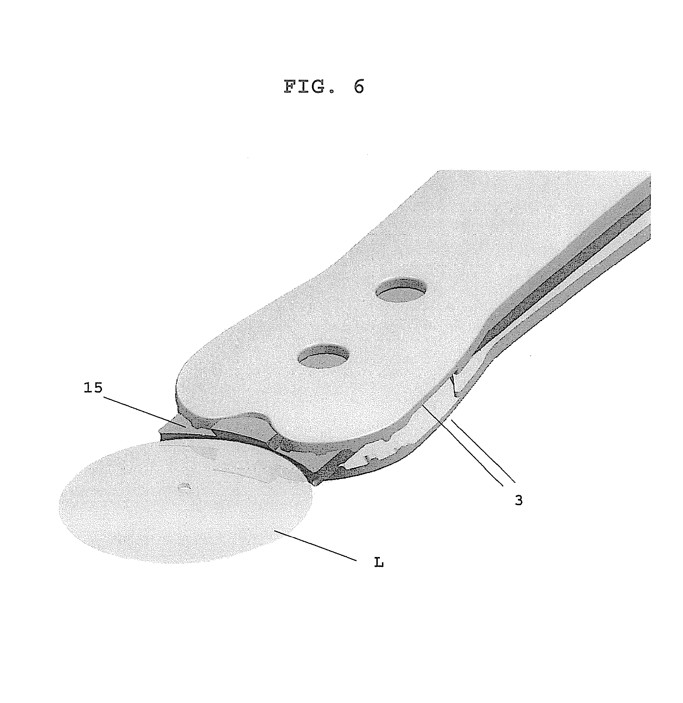

In **Fig. 6** ist gezeigt, wie eine Linse L aus der erfindungsgemässen erfindungsgemässen Pre-Load-Einheit freigesetzt wird. Der Schieber 15 ist so weit vorgeschoben, dass er in Kontakt zu der Linse L steht. Dadurch sind die blattartigen Einheiten 3 voneinander weg gespreizt. Die blattartigen Einheiten 3 können nun um einen definierten Betrag zurückgezogen werden, während der Schieber 15 die Linse L an dem vorher ausgerichteten Ort hält. Die Linse L ist nun freigesetzt, und der erfindungsgemässe Applikator kann entfernt werden.

## Patentansprüche

1. Pre-Load-Einheit (P) zur Einführung von Linsen in das Auge eines Menschen oder Tieres, umfassend
i) ein Gehäuse (2) mit Mitteln (12) zur, vorzugsweise verdrehsicheren, Befestigung der Einheit an einem Griffstück (7),
ii) ein Linsenaufnahmeteil (11), welches an oder in dem Gehäuse (2) angeordnet ist und einen aus dem Gehäuse (2) herausstehenden Abschnitt mit mindestens zwei, vorzugsweise zwei, getrennten blattartigen Einheiten (3) umfasst, welche zumindest an ihren vom Gehäuse (2) entfernten Enden miteinander in lösbarem Kontakt stehen und dort eine Kammer (14) zur Aufbewahrung einer optischen Linse (L) bilden,
iii) einen Schieber (15), welcher beweglich im Innern des Gehäuses (2) angeordnet ist und zwischen den blattartigen Einheiten (3) der Linsenaufnahmeeinheit (11) bewegt werden kann.

2. Pre-Load-Einheit (P) nach Anspruch 1, **dadurch gekennzeichnet, dass** die zwei blattartigen Einheiten (3) jeweils ein Loch (4) aufweisen, welche so angeordnet sind, dass die Löcher (4) direkt übereinander liegen und eine Öffnung durch das Zentrum der Kammer (14) zur Aufbewahrung einer optischen Linse (L) bilden.

3. Pre-Load-Einheit (P) nach Anspruch 1 oder 2, **dadurch gekennzeichnet, dass** die Begrenzung der Kammer zur Aufbewahrung einer optischen Linse (L) zumindest teilweise durch wellenförmige Abschnitte (13a-13d) gebildet wird, welche auf beiden blattartigen Einheiten (3) vorhanden und so angeordnet sind, dass die Abschnitte der einen blattartigen Einheit (3) mit den entsprechenden Abschnitten der anderen blattartigen Einheit (3) verzahnt sind.

4. Pre-Load-Einheit (P) nach einem der Ansprüche 1 bis 3, **dadurch gekennzeichnet, dass** zumindest eine blattartige Einheit (3) ein weiteres Loch (5) aufweist, welches oberhalb der Begrenzung der Kammer (14) zur Aufbewahrung einer optischen Linse (L) angeordnet ist.

5. Kit, umfassend eine Aufbewahrungseinheit (16) und eine Pre-Load-Einheit (P) gemäss einem der Ansprüche 1 bis 4 im Innern der Aufbewahrungseinheit (16), wobei die Aufbewahrungseinheit (16) aus einem wasserdichten Material besteht und mit einem Stopfen (17) wasserdicht verschliessbar ist.

6. Kit nach Anspruch 5, **dadurch gekennzeichnet, dass** der Innenraum der Aufbewahrungseinheit (16) mit physiologischer Kochsalzlösung gefüllt ist.

7. Kit nach Anspruch 5 oder 6, **dadurch gekennzeichnet, dass** die Pre-Load-Einheit (P) mit einer optischen Linse (L), vorzugsweise einer Intracorneallinse, aufbewahrt ist.

8. Kit nach einem der Ansprüche 5 bis 7, d**adurch gekennzeichnet, dass** zusätzlich ein Griffstück (7) zur Verbindung mit der Pre-Load-Einheit (P) bereitgestellt ist.

9. Applikator (1), umfassend eine Pre-Load-Einheit (P) gemäss einem der Ansprüche 1 bis 4 und ein Griffstück (7), wobei die Pre-Load-Einheit (P) und das Griffstück (7) derart miteinander verbunden sind, dass die Pre-Load-Einheit (P) innerhalb des Griffstücks (7) bewegt werden kann.

10. Applikator (1) nach Anspruch 9, **dadurch gekennzeichnet, dass** am Griffstück (7) Bedienelemente (8,9) zur Bewegung des Schiebers (15) und der restlichen Pre-Load-Einheit (P) vorgesehen sind.

11. Applikator (1) nach Anspruch 10, **dadurch gekennzeichnet, dass** ein erstes Bedienelement (8) zur Bewegung des Schiebers (15) und ein zweites Bedienelement (9) zur Bewegung der restlichen Pre-Load-Einheit (P) vorgesehen ist, wobei das zweite Bedienelement (9) nur bedient werden kann, wenn das erste Bedienelement (8) sich in einer Position befindet, in welcher der damit bewegte Schieber (15) maximal aus dem Gehäuse der Pre-Load-Einheit (P) herausgefahren ist.

12. Applikator nach einem der Ansprüche 9 bis 11, **dadurch gekennzeichnet, dass** die Pre-Load-Einheit (P) mit einer optischen Linse, vorzugsweise einer intracorneallinse, beschickt ist.

13. Verfahren zur Einführung einer optischen Linse (L), vorzugsweise einer Intracorneallinse, in das Auge eines Menschen oder Tieres, umfassend die Schritte
a) Positionieren des Applikators (1) gemäss einem der Ansprüche 9 bis 12 an der gewünschten Stelle des Auges, vorzugsweise in einer Tasche in der menschlichen Cornea, so dass das Zentrum der im Applikator (1) enthaltenen optischen Linse (L) auf der Sehachse des Auges liegt;
b) Vorschieben des Schiebers mittels eines ersten Bedienelements (8) am Griffstück (7), bis der Schieber (15) in Kontakt mit der Linse (L) kommt ohne diese zu bewegen, wobei gleichzeitig die blattartigen Einheiten (3) des Linsenaufnahmeteils (11) voneinander weg gespreizt werden;
c) Zurückziehen der restlichen Pre-Load-Einheit (P) aus dem Auge um einen definierten Betrag mittels eines zweiten Bedienelements (9) am Griffstück (7) unter gleichzeitiger Fixierung des Schiebers (15), wodurch die Linse (L) aus dem Applikator (1) freigesetzt wird.

14. Verfahren nach Anspruch 13, **dadurch gekennzeichnet, dass** die Positionierung des Applikators (1) in Schritt a) dadurch erfolgt, dass die Linse (L) mittels der Löcher (4) erfolgt, welche in den blattartigen Einheiten (3) so angeordnet sind, dass die Löcher (4) direkt übereinander liegen und eine Öffnung durch das Zentrum der Kammer (14) zur Aufbewahrung einer optischen Linse (L) bilden.

15. Verwendung einer Pre-Load-Einheit (P) gemäss einem der Ansprüche 1 bis 4 beziehungsweise eines Applikators (1) gemäss einem der Ansprüche 9 bis 12 zur Einführung einer optischen Linse (L), vorzugsweise einer Intracorneallinse, in das Auge eines Menschen oder Tieres, vorzugsweise in eine Tasche in der Cornea eines Menschen oder Tieres.

16. Verwendung einer Pre-Load-Einheit (P) gemäss einem der Ansprüche 1 bis 4 beziehungsweise eines Applikators (1) gemäss einem der Ansprüche 9 bis 12 zur Einführung einer Spenderhornhaut in das Auge eines Menschen oder Tieres.
